# EUROPÄISCHE PATENTSCHRIFT

(11) **EP 1 021 163 B1**
(45) Veröffentlichungstag und Bekanntmachung des Hinweises auf die Patenterteilung: **03.09.2003**
(21) Anmeldenummer: 98951394.0
(22) Anmeldetag: 15.09.1998
(51) Int. Cl.: A61K 7/48

(54) **KOSMETISCHE MITTEL**
COSMETIC PRODUCTS
PRODUITS COSMETIQUES

(30) Priorität: 24.09.1997 JP 25895197
(43) Veröffentlichungstag der Anmeldung: 26.07.2000
(73) Patentinhaber: Cognis Deutschland GmbH & Co. KG, 40589 Düsseldorf (DE)
(72) Erfinder: KAKUSHI, Doki, Tokyo 104-0042 (JP)
(86) Internationale Anmeldenummer: EP9805839
(87) Internationale Veröffentlichungsnummer: WO99015146

(56) Entgegenhaltungen:
- DD-A- 208 558
- DE-A- 2 024 051
- DE-A- 4 243 119
- DE-A- 19 525 108
- US-A- 3 934 003

## Beschreibung

### Gebiet der Erfindung

Die Erfindung befindet sich auf dem Gebiet der Hautkosmetik und betrifft Zubereitungen, insbesondere Hautreinigungsmittel zum Entfernen dekorativer Kosmetik, mit einem definierten Gehalt an nichtionischen Tensiden und gegebenenfalls Ölkörpern.

### Stand der Technik

Zur Reinigung der Haut von dekorativen Kosmetika auf Ölbasis wie etwa Lippenstift, Grundierungen, Wimperntusche oder Lidschatten wird üblicherweise eine zweistufige Verfahrensweise gewählt. Zunächst werden die fettigen Hautverschmutzungen mittels Massagecreme, Reinigungscreme, Reinigungsöl, Reinigungsgel und/oder anderen sich gut mit dekorativen Kosmetika verbindenden Gesichtswaschmitteln entfernt und in einem zweiten Schritt mit Seife oder Reinigungsschäumen auf der Haut verbliebene Fettrückstände, Talg und andere Verschmutzungen abgewaschen. Diese zweistufige Hautreinigung ist notwendig, weil bei der Anwendung von Ölkomponenten in größeren Mengen enthaltender Mittel (beispielsweise Massagecreme) die auf der Haut verbleibenden Ölkomponenten durch anschließendes Spülen mit kaltem oder lauwarmem Wasser nicht ausreichend entfernt werden können und der Haut daher kein Frischegefühl vermittelt werden kann. Wenn hingegen zur Hautreinigung lediglich Waschmittel wie Seife verwendet werden, ist eine Entfernung von Pigmenten und von anderen, mit Ölkomponenten umgebenen farbgebenden Substanzen nicht möglich. In den letzten Jahren wird jedoch von kosmetischen Mitteln einfache Anwendbarkeit gefordert und es werden Abschminkmittel gewünscht, die mit einer einmaligen Wäsche dekorative Kosmetika entfernen können und ein frisches, unfettiges Wäschegefühl zu vermitteln vermögen. Derartige einstufige Zubereitungen sind beispielsweise aus der **JP-A Sho 63/122618, JP-A Hei 04/005213 und JP-A Hei 07/215842** bekannt, wobei es sich jeweils um aus einer oder zwei Phasen aufgebaute Hautwaschmittel handelt, die bestimmte nichtionische Tenside in Kombination mit wasserlöslichen Substanzen enthalten. Weiterhin sind aus der **JP-A Hei 07/215840, der JP-A Hei 07/304630** sowie der **JP-A Hei 07/291831** schäumende Waschmittel bekannt, die auf bestimmten nichtionischen Tensiden und Aniontensiden aufgebaut sind. Diese einstufigen Hautwaschmittel können jedoch in zweierlei Hinsicht noch nicht als zufriedenstellend bezeichnet werden. Zum einen ist ihre Reinigungskraft gegenüber dekorativen Kosmetika, welche in den letzten Jahren immer höhere Anteile an Ölkomponenten aufweisen oder auch filmbildende Substanzen enthalten, gering; zum anderen ist der bei ihrer Anwendung vermittelte sensorische Eindruck z.B. was das Spreiten bzw. Gleiten der Mittel beim Auftrag auf die Haut anlangt, noch verbesserungsbedürftig.

In diesem Zusammenhang sei ferner auf die Deutsche Patentschrift **DE 2024051 C3** (Henkel) verwiesen, aus der die Verwendung von ethoxylierten Partialglyceriden als Rückfettungsmittel bekannt ist.

Die komplexe Aufgabe der Erfindung hat daher darin bestanden, kosmetische Mittel, speziell Hautreinigungsmittel zur Verfügung zu stellen, die die Haut in einem Schritt reinigen und dabei insbesondere dekorative Kosmetik, wie beispielsweise Make-up, Lidschatten, Rouge, Kajal, Mascara, Lippenstift und dergleichen, rückstandslos entfernen. Aus ästhetischen Gründen sollten die Zubereitungen einphasig, transparent und niedrigviskos sein und diese Eigenschaften auch während einer Temperaturlagerung nicht verlieren. Schließlich sollten die Mittel auf der Haut ein sensorisch angenehmes, leichtes Gefühl hinterlassen.

### Beschreibung der Erfindung

Gegenstand der Erfindung ist die Verwendung von Mischungen, enthaltend
(a) 20 bis 80, vorzugsweise 25 bis 35 Gew.-% ethoxylierte Partialglyceride und
(b) 0,1 bis 80, vorzugsweise 15 bis 75 Gew.-% Ölkörper
mit der Maßgabe, daß sich die Mengen gegebenenfalls mit Wasser und üblichen Hilfs- und Zusatzstoffen zu 100 Gew.-% ergänzen, zur Herstellung von Abschminkmitteln.

Überraschenderweise wurde gefunden, daß Mittel der genannten Art einphasig und transparent sind. Der Begriff der Einphasigkeit bezieht sich dabei darauf, daß es sich bei den Zusammensetzungen nicht um O/W- oder W/O-Emulsionen handelt, sondern um transparente bzw, semitransparente Zubereitungen. Sie weisen eine für die Anwendung ausreichende, jedoch nicht zu hohe Viskosität auf, die auch bei Temperaturlagerung erhalten bleibt. Die Erfindung schließt die Erkenntnis ein, daß die Zubereitungen mit Wasser leicht abzuspülen sind, dekorative Kosmetik - insbesondere Make-up, Wimperntusche und Lidschatten - in einem Schritt rückstandslos entfernen und dabei gleichzeitig ein angenehmes Hautgefühl vermitteln.

### Ethoxylierte Partialglyceride

Ethoxylierte Partialglyceride, also Anlagerungsprodukte von Ethylenoxid an Monoglyceride, Diglyceride und deren technische Gemische können herstellungsbedingt noch geringe Mengen Triglyceride enthalten. Die Partialglyceride folgen vorzugsweise der Formel (I), 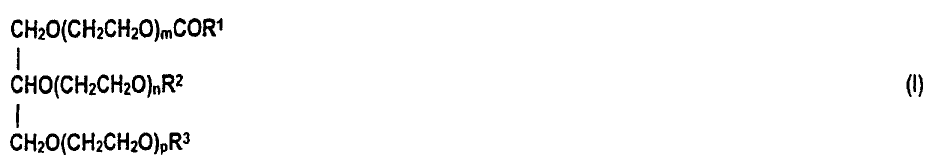 in der R¹CO für einen linearen oder verzweigten, gesättigten Acylrest mit 12 bis 18 Kohlenstoffatomen, R² und R³ unabhängig voneinander für R¹CO oder OH und die Summe (m+n+p) für Zahlen von 5 bis 10 steht, mit der Maßgabe, daß mindestens einer der beiden Reste R² und R³ OH bedeutet. Typische Beispiele sind Anlagerungsprodukte von 5 bis 10 Mol Ethylenoxid an Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische ethoxylierte Laurinsäureglyceride, Palmitinsäureglyceride, Stearinsäureglyceride, Isostearinsäureglyceride, Ölsäureglyceride, Behensäureglyceride und/oder Erucasäureglyceride mit etwa 7 Mol Ethylenoxid eingesetzt, welche einen Monoglyceridanteil im Bereich von 50 bis 95, vorzugsweise 60 bis 90 Gew.-% aufweisen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylengfycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

### Kosmetische Mittel

Die erfindungsgemäßen Zubereitungen, wie beispielsweise Cremes, Lotionen oder Salben, können femer als weitere Hilfs- und Zusatzstoffe milde Tenside, Emulgatoren, Überfettungsmittel, Perlglanzwachse, Stabilisatoren, Konsistenzgeber, Verdickungsmittel, Polymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen enthalten, Die Mittel eignen sich nicht nur zur Reinigung der Haut, sondern können diese auch pflegen oder schützen. Mögliche Ausführungsformen der Erfindung umfassen daher auch die Verwendung der Zubereitungen zur Herstellung von Abschmink- und Sonnenschutzmitteln.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS als** Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im. Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Periglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phy-tantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copoly-merisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 24,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P.Finkel in **SÖFW-Journal 122, 543 (1996)** zu entnehmen.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsem (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, ∝-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Es wurden bei 40°C 25 Gew.-%ige Lösungen verschiedener nichtionischer Tenside zubereitet und dann sowohl das Aussehen der Mittel als auch ihre Fähigkeit, dekorative Kosmetika zu entfernen sowie ihre Abspülbarkeit mit Wasser beurteilt. Die Bewertung erfolgte subjektiv auf einer Skala von 1 bis 5 : (1) = sehr gut, transparent; (2) = gut; (3) = befriedigend, leicht getrübt; (4) = ausreichend, trüb; (5) mangelhaft, stark getrübt. Tabelle 1 zeigt, daß ethoxylierte Partialglyceride den anderen bei Zimmertemperatur flüssigen nichtionischen Tensiden überlegen sind. Die Lösungen sind transparent und zeigen eine bessere Reinigungskraft und eine bessere Abspülbarkeit. Die in den Beispielen gemachten Angaben zu Dosierungen verstehen sich als Gew.-%. Die Beispiele 1 bis 5 sind erfindungsgemäß, die Beispiele V1 bis V5 dienen zum Vergleich.

**Tabelle 1**

| **Wäßrige Niotensidlösungen** | | | | | | | | | | |
|---|---|---|---|---|---|---|---|---|---|---|
| **Niotensid / Bewertung** | **1** | **2** | **3** | **4** | **5** | **V1** | **V2** | **V3** | **V4** | **V5** |
| PEG-7 Glyceryl Laurate | 25 | - | - | - | - | - | - | - | - | - |
| PEG-7 Glyceryl Cocoate | - | 25 | - | - | - | - | - | - | - | - |
| PEG-10 Glyceryl Cocoate | - | - | 25 | - | - | - | - | - | - | - |
| PEG-10 Glyceryl Isostearate | - | - | - | 25 | - | - | - | - | - | - |
| PEG-20 Glyceryl Cocoate | - | - | - | - | 25 | - | - | - | - | - |
| PEG-20 Hydrogenated Castor Oil | - | - | - | - | - | 25 | - | - | - | - |
| PEG-40 Sorbitan Monooleate | - | - | - | - | - | - | 25 | - | - | - |
| PEG-20 Sorbitan Monostearate | - | - | - | - | - | - | - | 25 | - | - |
| PEG-30 Sorbitan Tetraoleate | - | - | - | - | - | - | - | - | 25 | - |
| PEG-12 Monostearate | - | - | - | - | - | - | - | - | - | 25 |
| Wasser | ad 100 | | | | | | | | | |
| ***Erscheinungsbild (20°C)*** | 1 | 1 | 1 | 1 | 1 | 4 | 1 | 4 | 4 | 1 |
| ***Reinigungsvermögen*** | 1 | 1 | 1 | 2 | 2 | 5 | 3 | 5 | 5 | 3 |
| **Abspülbarkeit** | 1 | 1 | 1 | 2 | 2 | 5 | 3 | 5 | 5 | 2 |

Die In Tabelle 2 aufgeführten Inhaltsstoffe wurden bei 70 °C miteinander vermischt und dann unter Rühren auf etwa 20°C abgekühlt. Es wurden transparente Gesichtsreiniger erhalten, die wiederum auf der Skala gemäß Tabelle 1 bewertet wurden. Die erfindungsgemäßen Mittel sind transparent, zeichnen sich durch ein angenehmes Anwendungsgefühl aus und weisen eine hervorragende Reinigungskraft und Abspülbarkeit auf. Die Beispiele 6 bis 11 sind erfindungsgemäß, die Beispiele V6 bis V9 dienen zum Vergleich.

**Tabelle 2**

| **Kosmetische Mittel** | | | | | | | | | | |
|---|---|---|---|---|---|---|---|---|---|---|
| **Niotensid/Bewertung** | **6** | **7** | **8** | **9** | **10** | **11** | **V6** | **V7** | **V8** | **V9** |
| PEG-7 Glyceryl Cocoate | 25 | 50 | 50 | 40 | 30 | 20 | 5 | 10 | 15 | 10 |
| 2-Ethylhexylic Glycerides | 5 | - | - | - | - | - | - | - | - | - |
| Octyldodecanol | - | 20 | - | - | - | - | - | - | - | - |
| Paraffin, flüssig | - | - | 50 | 60 | 70 | 80 | - | - | - | 90 |
| Wasser | ad 100 | | | | | | | | | |
| ***Erscheinungsbild (20°C)*** | 1 | 1 | 1 | 1 | 1 | 1 | 3 | 3 | 3 | 1 |
| ***Reinigungsvermögen*** | 1 | 1 | 1 | 1 | 1 | 1 | 4 | 3 | 1 | 2 |
| ***Abspülbarkeit*** | 1 | 1 | 1 | 1 | 1 | 2 | 4 | 3 | 3 | 3 |

**Formulierungsbeispiel.** Die Inhaltsstoffe der im folgenden angegebenen Rezeptur wurden in der Wärme aufgeschmolzen und verrührt. Nach dem Abkühlen wurden transparente Zubereitungen erhalten. Da es sich bei dem Gesichtsreiniger um ein flüssiges Mittel handelt, ermöglicht es ein Dispergieren bzw. Lösen auch von tief in der Haut sitzendem Schmutz. Nach dem Reinigen läßt sich die Zubereitung leicht mit Wasser abspülen und die Haut fühlt sich erfrischt an. Die Mengenangaben verstehen sich als Gew.-%, Wasser ad 100 Gew.-%.

| **Gesichtsreiniger** | |
|---|---|
| PEG-7 Glyceryl Laurate | 25,0 |
| 2-Ethyhexylic Glycerides | 5,0 |
| 1,3-Butandiol | 7,0 |
| Parabene | 0,1 |

## Patentansprüche

1. Verwendung von Mischungen, enthaltend
(a) 20 bis 80 Gew.-% ethoxylierte Partialglyceride der Formel **(I)** 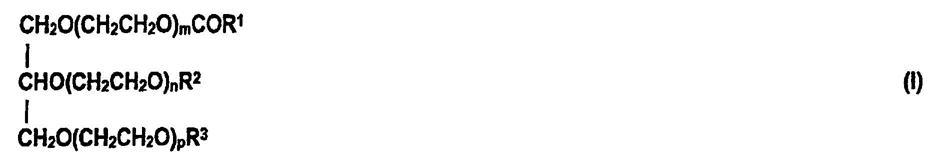 in der R¹CO für einen gesättigten Acylrest mit 12 bis 18 Kohlenstoffatomen, R² und R³ unabhängig voneinander für R¹CO oder OH und die Summe (m+n+p) für Zahlen von 5 bis 10 steht, mit der Maßgabe, daß mindestens einer der beiden Reste R² und R³OH bedeutet, und
(b) 0,1 bis 80 Gew.-% Ölkörper
mit der Maßgabe, daß sich die Mengen gegebenenfalls mit Wasser und üblichen Hilfs- und Zusatzstoffen zu 100 Gew.-% ergänzen, zur Herstellung von Abschminkmitteln.

## Claims

1. The use of mixtures containing
(a) 20 to 80% by weight of ethoxylated partial glycerides corresponding to formula **(I):** 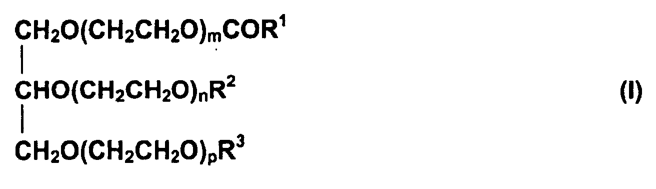 where R¹CO is a saturated acyl group containing 12 to 18 carbon atoms, R² and R³ independently of one another have the same meaning as R¹CO or represent OH and the sum (m+n+p) is a number of 5 to 10, with the proviso that at least one of the two substituents R² and R³ is OH,
and
(b) 0.1 to 80% by weight of oil components,
with the proviso that the quantities add up to 100% by weight, optionally with water and typical auxiliaries and additives,
for the production of make-up remover.

## Revendications

1. Utilisation de mélanges contenant,
(a) de 20 à 80 % en poids de glycérides partiels éthoxylés de formule (I), 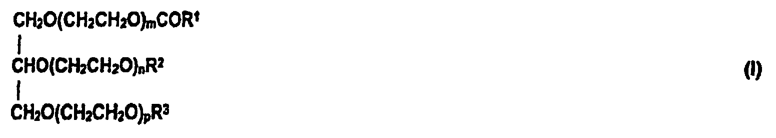 dans laquelle R¹CO représente un reste acyle saturé ayant de 12 à 18 atomes de carbone, R² et R³ indépendamment l'un de l'autre, représente R¹CO ou OH et la somme (m+n+p) représente des nombres allant de 5 à 10, avec la précision qu'au moins un des deux restes R² et R³, signifie OH, et
(b) de 0,1 à 80 % en poids de composé huileux avec la précision que les quantités se complètent le cas échéant avec l'eau et des adjuvants et des additifs usuels à 100 % en poids, en vue de la production d'agents démaquillants.
